(19) **Europäisches Patentamt European Patent Office Office européen des brevets**

(11) **EP 4 778 456 A1**

(12) **EUROPEAN PATENT APPLICATION**

(43) Date of publication:
**22.07.2026 Bulletin 2026/30**

(21) Application number: **26151413.7**

(22) Date of filing: **12.01.2026**

(51) International Patent Classification (IPC):
**A61B 5/0538** (2021.01)    **A61B 5/287** (2021.01)
**A61B 5/06** (2006.01)    **A61B 5/00** (2006.01)

(52) Cooperative Patent Classification (CPC):
**A61B 5/287; A61B 5/0538; A61B 5/6858;**
**A61B 5/6885;** A61B 5/068

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **16.01.2025 US 202563745980 P**

(71) Applicant: **St. Jude Medical, Cardiology Division, Inc.**
**St. Paul, MN 55117 (US)**

(72) Inventors:
• **SCHWEITZER, Jeffrey A.**
**St. Paul, 55108 (US)**
• **NGUYEN, Tri Minh**
**Everett, 02149 (US)**
• **DAHLEN, Travis**
**Forest Lake, 55025 (US)**

(74) Representative: **Mathys & Squire**
**32 London Bridge Street**
**The Shard**
**London SE1 9SG (GB)**

(54) **SYSTEMS AND METHODS OF UTILIZING SENSE CHANNELS CONNECTED TO A PLURALITY OF ELECTRODES**

(57)    A computer-implemented method and system. The method includes receiving, via a first sense channel, a first electrode impedance signal associated with a first electrode. The method further includes receiving, via a second sense channel, a second electrode impedance signal associated with at least a second and third electrode, wherein the second and third electrodes are electrically connected to each other but not to the first electrode. The method further assesses tissue contact/proximity based on the first electrode impedance signal and the second electrode impedance signal.

**FIG. 1**

EP 4 778 456 A1

**Description**

**BACKGROUND**

Intracardiac electrophysiology devices typically include a catheter having a proximal end and a distal end that includes a plurality of electrodes. Signals sensed by the plurality of electrodes are utilized by electrophysiology systems to perform a number of functions, including determining the location of the electrodes within the patient's body and sensing electrograms associated with adjacent cardiac tissue. This information can be utilized to understand electrical activity of the heart and identify the origins and pathways of abnormal heart rhythms such as arrythmias.

Software, such as the Ensite X cardiac mapping system is an example of one such system that may be utilized with a plurality of different catheter types to provide EP mapping. Each different type of catheter device has a different configuration of electrodes. For example, in some devices at least some electrodes are connected individually to sense channels and at least some electrodes are connected together such that a sense channel is connected to a plurality of electrodes.

It would be beneficial to be able to utilize information from sense channels connected to a plurality of electrodes in addition to information from sense channels connected to single electrodes in providing additional functionality for connected devices.

**SUMMARY**

The invention is directed to methods and system of utilizing information received from a sense channel connected to more than one electrode. One aspect is directed to utilizing impedance signals measured by the electrodes, including impedance signals measured by sense channels connected to a plurality of electrodes, to determine tissue contact/proximity. A second aspect is directed to detecting a deployment status of the distal end of the catheter based on localization signals received measured by the sense channels, including sense channels connected to a plurality of electrodes. A third aspect is directed to verifying that the sense channels are properly connected (in either hardware or software) to generate electrogram signals based on signals received from electrodes located on the same spline. This method relies on both impedance signals measured by each of the sense channels and locations signals measured by each of the sense channels, including sense channels connected to a plurality of electrodes.

In some aspects, the techniques described herein relate to a computer-implemented method of assessing tissue contact for a plurality of electrodes located on a medical device, the method including: receiving, via a first sense channel, a first electrode impedance signal associated with a first electrode; receiving, via a second sense channel, a second electrode impedance signal associated with at least a second and third electrode, wherein the second and third electrodes are electrically connected to each other but not to the first electrode; and assessing tissue contact/proximity based on the first electrode impedance signal and the second electrode impedance signal.

In some aspects, the techniques described herein relate to a computer-implemented method, wherein assessing tissue contact/proximity further includes: combining the first electrode impedance signal and the second electrode impedance signal into an aggregated electrode impedance signal; and comparing the aggregated electrode impedance signal to a baseline value to determine tissue contact/proximity.

In some aspects, the techniques described herein relate to a computer-implemented method, wherein comparing the aggregated electrode impedance signal to a baseline value includes comparing the aggregated electrode impedance signal to a threshold value, wherein tissue contact is detected if the aggregated electrode impedance signal is greater than the threshold value.

In some aspects, the techniques described herein relate to a computer-implemented method, wherein the threshold value is selected as representative of both the first electrode and at least one of the second and third electrodes being in contact with tissue.

In some aspects, the techniques described herein relate to a computer-implemented method, wherein the threshold value is selected as representative of at least one of the first electrode or the second and third electrodes being in contact with tissue.

In some aspects, the techniques described herein relate to a computer-implemented method, wherein combining the first electrode impedance signal and the second electrode impedance signal into an aggregated electrode impedance signal includes scaling the second electrode impedance signal to a value representative of a single electrode impedance signal.

In some aspects, the techniques described herein relate to a computer-implemented method, wherein scaling the second electrode impedance signal includes multiplying the second electrode impedance signal by a number of electrodes connected to the second sense channel.

In some aspects, the techniques described herein relate to a computer-implemented method, wherein combining the first electrode impedance signal and the second electrode impedance signal further includes adding the first electrode impedance signal to the scaled second electrode impedance signal and dividing by a number of sense channels.

In some aspects, the techniques described herein relate to a computer-implemented method, wherein assessing tissue contact/proximity further includes: comparing the first electrode impedance signal to a first baseline value to detect a first change in impedance; comparing the second electrode impedance signal to a second baseline value to detect a second change in impedance; scaling the second change in impedance to a value representative of single electrode impedance measurements; combining the first change in impedance and the scaled second change in impedance; and assessing tissue contact/proximity based on a comparison of the combined change in impedance to a threshold value.

In some aspects, the techniques described herein relate to a computer-implemented method, wherein scaling the second change in impedance includes multiplying the second change in impedance by a number of electrodes connected to the second sense channel.

In some aspects, the techniques described herein relate to a computer-implemented method, wherein the first, second and third electrodes are located on a single spline.

In some aspects, the techniques described herein relate to a computer-implemented method, further including generating an output indicating tissue contact/proximity of the spline based on the measured first electrode impedance signal and the measured second electrode impedance signal.

In some aspects, the techniques described herein relate to a computer-implemented method of determining a deployment status of an electrode assembly located at a distal end of a catheter having a plurality of splines and a plurality of electrodes on each spline, the method including: calculating with respect to each spline a position of a first electrode located on each spline based on a first impedance-based localization signal received on a first sense channel; calculating with respect to each spline a composite position of at least second and third electrodes located on each spline based on a second impedance-based localization signal received on a second sense channel; and determining a deployment status of the electrode assembly based on the position of the first electrode and the composite position of the at least second and third electrodes.

In some aspects, the techniques described herein relate to a computer-implemented method, wherein the deployment status of the electrode assembly includes a low profile status, a basket profile status, and a flower profile status.

In some aspects, the techniques described herein relate to a computer-implemented method, wherein determining the deployment status of the electrode assembly further includes: calculating, for each spline, vectors between the position of the first electrode and the composite position of at least the second and third electrodes for each spline; and determining a deployment status based on the vectors calculated for each of the plurality of splines.

In some aspects, the techniques described herein relate to a computer-implemented method, further including: summing the vectors calculated for each of the plurality of splines to generate a summed vector; comparing the summed vector to a unit vector; and determining deployment status of the electrode assembly based on the comparison of the summed vector to the unit vector.

In some aspects, the techniques described herein relate to a computer-implemented method, wherein the unit vector is a unit normal vector of a plane defined to best fit the position of the first electrodes associated with each spline, a plane defined to best fit the composition position of the second and third electrodes associated with each spline, or a plane defined to best fit both the position of the first electrodes and the composite position of the second and third electrodes associated with each spline.

In some aspects, the techniques described herein relate to a computer-implemented method, wherein the summed vector is compared to the unit vector using a dot product function, wherein the dot product output is utilized to determine the deployment status of the distal end of the catheter.

In some aspects, the techniques described herein relate to a computer-implemented method, further including: calculating second vectors between the position of the first electrodes on adjacent splines; and determining a deployment status based on the vectors calculated for each of the plurality of splines and the second vectors calculated between the position of the first electrodes on adjacent splines.

In some aspects, the techniques described herein relate to a computer-implemented method of confirming proper pin jack connection order for connection to a plurality of electrodes located at a distal end of a medical device, the method including: measuring electrode impedance signals associated with each of a plurality of sense channels; determining a position/composite position associated with each of the plurality of sense channels based on impedance-based localization signals measured by each sense channel, the position/composite positions corresponding with electrodes or groups of electrodes connected to each of the plurality of sense channels; organizing the plurality of sense channels into a first group of sense channels and a second group of sense channels based on the measured electrode impedance signals; pairing each sense channel in the first group with the sense channel in the second group that is closest in proximity based on the determined positions/composite positions of each of the plurality of sense channels; and measuring bipolar electrogram (egm) signals based on the pairing of each sense channel in the first group with a sense channel in the second group.

In some aspects, the techniques described herein relate to a computer-implemented method, wherein at least some of the sense channels are connected to single electrodes located on an electrode assembly located at the distal end of a medical device and at least some of the sense channels are connected to a plurality of electrodes located on the electrode assembly located at the distal end of the medical device.

In some aspects, the techniques described herein relate to a computer-implemented method, wherein the measured electrode impedance signals associated with sense channels connected to single electrodes are greater than the measured electrode impedance signals associated with sense channels connected to a plurality of electrodes.

In some aspects, the techniques described herein relate to a computer-implemented method, wherein the electrode assembly located at the distal end of the medical device is included of a plurality of splines, each spline having an plurality of electrodes, wherein at least some of the sense channels are connected to a single electrode on each spline and at least some of the channels are connected to a plurality of electrodes on the same spline.

In some aspects, a computer program product comprising instructions which, when the program is executed by a computer, cause the computer to carry out the methods described above.

In some aspects, a computer-readable medium storing instructions that, when executed by a processor, cause the processor to perform a method of assessing tissue contact for a plurality of electrodes located on a medical device. The method includes measuring, using a first sense channel, a first electrode impedance signal associated with a first electrode, measuring, using a second sense channel, a second electrode impedance signal associated with at least a second and third electrode, wherein the second and third electrodes are electrically connected to each other but not to the first electrode, and assessing tissue contact/proximity based on the first electrode impedance signal and the second electrode impedance signal.

According to some aspects, a computer-readable medium storing instructions that, when executed by a processor, cause the processor to perform a method of assessing tissue contact for a plurality of electrodes located on a medical device. The method includes calculating with respect to each spline a position of a first electrode located on each spline based on a first impedance-based localization signal received on a first sense channel, calculating with respect to each spline a composite position of at least second and third electrodes located on each spline based on a second impedance-based localization signal received on a second sense channel, and determining a deployment status of the electrode assembly based on the position of the first electrode and the composite position of the at least second and third electrodes.

According to some aspects, a computer-readable medium storing instructions that, when executed by a processor, cause the processor to perform a method of assessing tissue contact for a plurality of electrodes located on a medical device. The method includes measuring electrode impedance signals associated with each of a plurality of sense channels, determining a position/composite position associated with each of the plurality of sense channels based on impedance-based localization signals measured by each sense channel, the position/composite positions corresponding with electrodes or groups of electrodes connected to each of the plurality of sense channels, and organizing the plurality of sense channels into a first group of sense channels and a second group of sense channels based on the measured electrode impedance signals. The method further includes pairing each sense channel in the first group with the sense channel in the second group that is closest in proximity based on the determined positions/composite positions of each of the plurality of sense channels. Bipolar electrogram (EGM) signals are measured based on the pairing of each sense channel in the first group with a sense channel in the second group.

According to some aspects, a_system for assessing tissue contact for a plurality of electrodes located on a medical device, the system comprising a tissue contact module configured to: measure, using a first sense channel, a first electrode impedance signal associated with a first electrode; measure, using a second sense channel, a second electrode impedance signal associated with at least a second and third electrode, wherein the second and third electrodes are electrically connected to each other but not to the first electrode; and assess tissue contact/proximity based on the first electrode impedance signal and the second electrode impedance signal.

In some aspects, the tissue contact module is configured to assess tissue contact/proximity by: combining the first electrode impedance signal and the second electrode impedance signal into an aggregated electrode impedance signal; and comparing the aggregated electrode impedance signal to a baseline value to determine tissue contact/proximity.

In some aspects, comparing the aggregated electrode impedance signal to a baseline value includes comparing the aggregated electrode impedance signal to a threshold value, wherein tissue contact is detected if the aggregated electrode impedance signal is greater than the threshold value, optionally (i) wherein the threshold value is selected as representative of both the first electrode and at least one of the second and third electrodes being in contact with tissue, or (ii) wherein the threshold value is selected as representative of at least one of the first electrode or the second and third electrodes being in contact with tissue.

In some aspects, combining the first electrode impedance signal and the second electrode impedance signal into an aggregated electrode impedance signal includes scaling the second electrode impedance signal to a value representative of a single electrode impedance signal, optionally wherein scaling the second electrode impedance signal includes multiplying the second electrode impedance signal by a number of electrodes connected to the second sense channel, optionally wherein combining the first electrode impedance signal and the second electrode impedance signal further includes adding the first electrode impedance signal to the scaled second electrode impedance signal and dividing by a number of sense channels.

In some aspects, assessing tissue contact/-proximity further includes: comparing the first electrode impedance signal to a first baseline value to detect a first change in impedance; comparing the second electrode impedance signal to a second baseline value to detect a second change in impedance; scaling the second change in impedance to a value representative of single electrode impedance measurements; combining the first change in impedance and the scaled second change in impedance; and assessing tissue contact/proximity based on a comparison of the combined change in impedance to a threshold value, optionally wherein scaling the second change in impedance includes multiplying the second change in impedance by a number of electrodes connected to the second sense channel.

In some aspects, the first, second and third electrodes are located on a single spline, optionally further including generating an output indicating tissue contact/proximity of the spline based on the measured first electrode impedance signal and the measured second electrode impedance signal.

## BRIEF DESCRIPTION OF THE DRAWINGS

**FIG.** 1 is a diagrammatic depiction of a system including a medical device for insertion within a patient, the system configured to interact with electrodes located on a distal end of the medical device.

**FIG. 2A, 2B** are isometric views of a distal end of the medical device in first and second configurations, the distal end of the medical device including a plurality of splines and a plurality of electrodes on each spline.

**FIG.** 3 is a block diagram of a first sensing channel connected to a first electrode and a second sensing channel connected to a plurality of electrodes.

**FIG.** 4 is a flowchart illustrating steps implemented by the tissue contact module to assess tissue contact/proximity based on signals received from the first sensing channel and the second sensing channel.

**FIGS. 5A, 5B** are flowcharts illustrating steps implemented by the tissue contact module to assess tissue contact/proximity based on local electrode impedance signals received from the first sensing channel and the second sensing channel.

**FIG.** 6 is a flowchart illustrating steps implemented by the shape detection module to detect the shape of the distal end of the medical device based on impedance-based localization signals received from a plurality of first channels and second channels.

**FIG.** 7 is a flowchart illustrating in additional detail steps implemented by the shape detection module to detect the shape of the distal end of the medical device based on impedance-based localization signals received from a plurality of first channels and second channels.

**FIG. 8A, 8B** are isometric views of a distal end of the medical device in first and second configurations and illustrating vectors created by the shape detection module based on impedance-based localization signals received from a plurality of first channels and second channels.

**FIG.** 9 is a graph illustrating values generated based on the steps shown in FIG. 7 and corresponding shape of the distal end of the medical device.

**FIG. 10** is a flowchart illustrating steps implemented by the pin jack detection module to pair sense channels as required to generate bipolar electrogram pairs.

## DETAILED DESCRIPTION

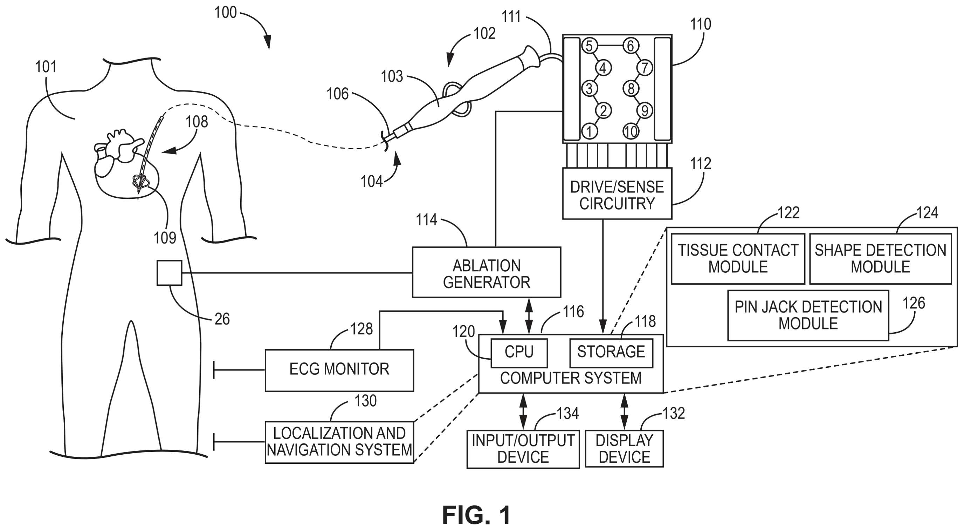

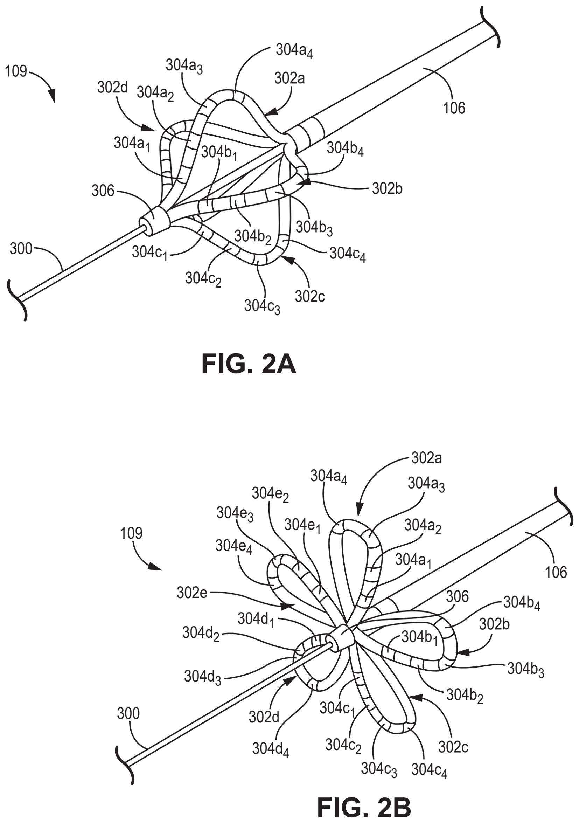

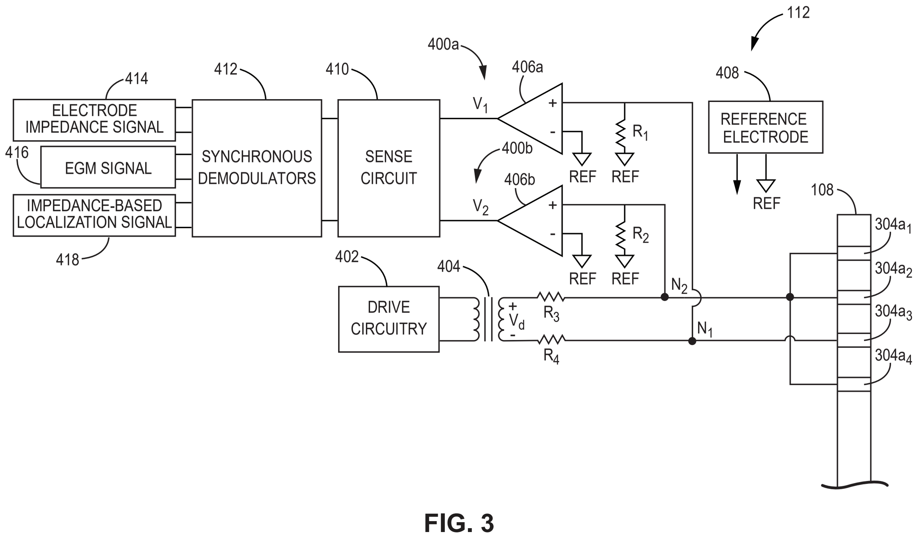

**FIG. 1** is a diagrammatic depiction of a system 100 including a medical device 102 for insertion within a patient. The medical device 102 includes handle 103 and a shaft 106 having a proximal end 104 and a distal end 108. An electrode assembly 109 is located at the distal end 108 of the shaft 106 includes a plurality of electrodes (shown in more detail in **FIGS. 2A**, **2B**). In some examples, the electrode assembly 109 is a basket comprised of a plurality of individual splines, each spline including a plurality of electrodes. A plurality of wires connect the electrodes to the handle 103. A cable 111 is connected to the handle 103 and includes a plurality of pins for interfacing the handle 103 with an input socket 110. In this way, electrical signals sensed by the plurality of electrodes are communicated to the handle 103 and via the input socket 110 to drive/sense circuitry 112 and computer system 116. As described in more detail below, in some examples subsets of electrodes included as part of the electrode assembly 109 are connected together (i.e., a plurality of electrodes are connected in parallel with one another) while other electrodes are individually connected. As a result, a subset of the plurality of pins associated with the cable 111 are each connected to a plurality of electrodes while another subset of the plurality of pins associated with the cable 111 are each connected to individual electrodes. Each of the plurality of pins is connected to a sense channel associated with drive/-sense circuitry 112 (discussed in more detail in **FIG. 3**). As described in more detail below, despite a subset of the channels being connected to a plurality of electrodes and a subset of channels being connected to individual electrodes, useful information can be gleaned from both and utilized for various functions, including tissue contact/-proximity, shape detection of the electrode assembly 109, and/or pin jack verification (i.e., ensuring the plurality of pins associated with cable 111 are connected to the proper input sockets 110).

Computer system 116 is configured to receive signals provided by drive/sense circuitry 112, communicate with ablation generator 114, ECG monitor 128, input/output device 134, and display device 132. In addition, computer system 116 includes a central processing unit (CPU) 120 and memory/storage device 118, wherein the CPU 120 executes instructions stored by the storage device 118 to implement various functions and operations described herein, including a tissue contact module 122, a shape detection module 124, a pin jack detection module 126, and localization and navigation system 130. Sensed signals received from the drive/sense circuitry 112 may include one or more of electrode impedance signals (i.e., tissue impedance signals), electrogram (EGM) signals, and/or impedance-based localization signals. Each of these signals may be monitored with respect to both the sense channels connected to a single electrode and the sense channels connected to a plurality of electrodes. The computer system 116 utilizes the signals received from the drive/sense circuitry 112 for a variety of functions, including localization/navigation of the electrode assembly 109 through the patient's body 101, tissue contact/proximity detection of the electrode assembly 109 with adjacent tissue, pin jack verification (ensuring the plurality of pins associated with the cable 111 are connected to the correct input sockets 110), and shape detection (e.g., for an electrode assembly 109 that includes a plurality of splines, the plurality of splines may be placed into a low profile shape, a basket geometry, or a flower geometry). Each of these functions utilizes information from sense channels connected to a single electrode and information from sense channels connected to a plurality of electrodes, as described in more detail below.

Outputs generated by the computer system 116 may be displayed to a technician/user via display device 132. Likewise, a technician/user may provide inputs to the computer system via input/output device 134.

**FIG. 2A, 2B** are isometric views of an electrode assembly 109 of the medical device 102 in first and second configurations. The electrode assembly 109 is located at a distal end of the shaft 106 and in this embodiment is guided by a guidewire 300 that extends within the inner portion of the electrode assembly and through distal cap 306. The electrode assembly 109 consists of a plurality of splines 302a, 302b, 302c, 302d, and 302e (collectively, "splines 302"), each spline 302 connected on a proximal end to the shaft 106 and on a distal end to the distal cap 306. The electrode assembly 109 includes a plurality of electrodes located on each spline 302. For example, spline 302a includes electrodes $304a_1$, $304a_2$, $304a_3$, and $304a_4$, spline 302b includes electrodes $304b_i$, $304b_2$, $304b_3$, and $304b_4$, spline 302c includes electrodes $304c_1$, $304c_2$, $304c_3$, and $304c_4$, spline 302d includes electrodes $304d_i$, $304d_2$, $304d_3$, and $304d_4$, and spline 302e includes electrodes $304e_1$, $304e_2$, $304e_3$, and $304e_4$. In other examples, each of the plurality of splines may include fewer or greater numbers of electrodes.

In one example, the first, second and fourth electrode on each spline is connected together. For example, with respect to spline 302a, electrodes $304a_i$,

$304a_2$, and $304a_4$ are connected to a single sense channel, while electrode $304a_3$ is connected to an individual sense channel. In this example, each of the plurality of electrodes on the other splines 302b, 302c, 302d, and 302e are connected in the same configuration. This configuration of electrodes utilizes ten total sense channels. The first subset of sense channels is each connected to a plurality of electrodes. For example, the first subset of sense channels would include a first channel connected to electrodes $304a_1$, $304a_2$, and $304a_4$, a second channel connected to electrodes $304b_1$, $304b_2$, and $304b_4$, a third channel connected to electrodes $304c_1$, $304c_2$, and $304c_4$, a fourth channel connected to electrodes $304d_1$, $304d_2$, and $304d_4$, and a fifth channel connected to electrodes $304e_1$, $304e_2$, and $304e_4$. The second subset of sense channels is each connected to an individual electrode. For example, the sixth channel is connected to electrode $304a_3$, the seventh channel is connected to electrode $304b_3$, the eighth channel is connected to electrode $304c_3$, the ninth channel is connected to electrode $304d_3$, the tenth channel is connected to electrode $304e_3$. In this example, a total of ten sense channels are utilized, five connected to a plurality of electrodes and five connected to individual electrodes.

In the embodiment shown in **FIG. 2A**, electrode assembly 109 is in a basket configuration in which the distal cap 306 is separated from a proximal end of the electrode assembly 109 by some distance. In the embodiment shown in **FIG. 2B**, electrode assembly 109 is in a flower configuration in which the distal cap 306 is located adjacent to the proximal end of the electrode assembly 109. For the type of electrode assembly 109 shown in **FIGS. 2A, 2B,** another type of configuration is described as a low-profile configuration in which the distal cap 306 is located as far as possible from the proximal end and the splines 302a-302e are positioned in a low-profile to allow the electrode assembly 109 to fit within a sheath. As described in more detail below, methods of detecting the configuration of the electrode assembly 109 may be beneficial to a technician/physician.

**FIG. 3** is a block diagram of drive/sense circuitry 112, illustrating a first sensing channel 400a connected to a first electrode $304a_3$ and a second sensing channel 400b connected to a plurality of electrodes that includes electrodes 304ai, $304a_2$, and $304a_4$. In this example, the drive/sense circuitry 112 includes drive circuitry 402 configured to provide via transformer 404 a constant current source to the plurality of electrodes, with node Ni connected to electrode $304a_3$ and node $N_2$ connected to plurality of electrodes $304a_1$, $304a_2$, and $304a_4$.

The first sense channel 400a includes a first operational amplifier 406a having a first input (e.g., non-inverting input) connected to electrode $304a_3$ and a second input (e.g., inverting input) connected to a reference node (e.g., reference electrode 408). In some examples, the reference electrode 408 is located on the same catheter as electrode assembly 109 (e.g., reference electrode may be a shaft electrode), on a separate catheter, or is attached as a surface patch electrode on the skin of the patient. The second sense channel 400b includes a second operational amplifier 406b having a first input (e.g., non-inverting input) connected to a plurality of electrodes, including in this example electrodes $304a_1$, $304a_2$, and $304a_4$ and a second input (e.g., inverting input) connected to the reference node (e.g., reference electrode 408). In this way, the first sense channel receives or measures a voltage signal sensed by electrode $304a_3$ and the second sense channel receives or measures a voltage signal sensed by electrodes $304a_1$, $304a_2$, and $304a_4$. As illustrated in FIG. 3, electrodes $304a_1$, $304a_2$, and $304a_4$ are connected in parallel with one another and the sensed voltage reflects this connection configuration.

The output generated by first and second op amps 406a, 406b is provided to sense circuit 412 and synchronous demodulator 412 to filter and demodulate signals sensed on each channel. In the example provided in FIG. 3, each sense channel 400a and 400b is capable of receiving or measuring an electrode impedance signal 414, an electrogram (EGM) signal 416, and an impedance-based localization signal 418. Synchronous demodulator 412 acts to filter or separate particular signals from one another as well as from background noise signals based on knowledge of the frequency of each signal. For example, the electrode impedance signal is measured in response to the drive current provided by the drive circuit 402 at a given frequency, and synchronous demodulator filters the measured response to the drive current by multiplying the received signal - including other signals and background noise - by a signal have the same frequency and phase as the drive current (or with a given phase offset). It should be noted that additional components not shown here may be required to process the monitored signals, such as analog-to-digital converters (ADC), switches, etc.

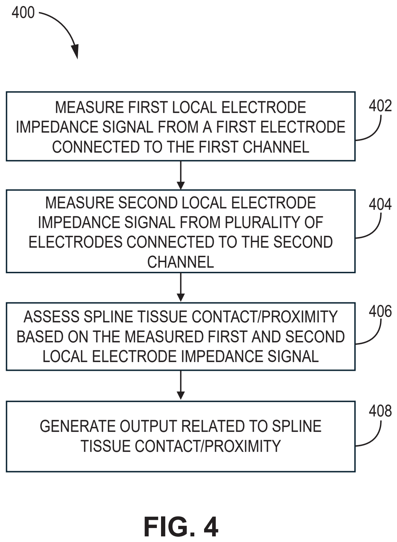

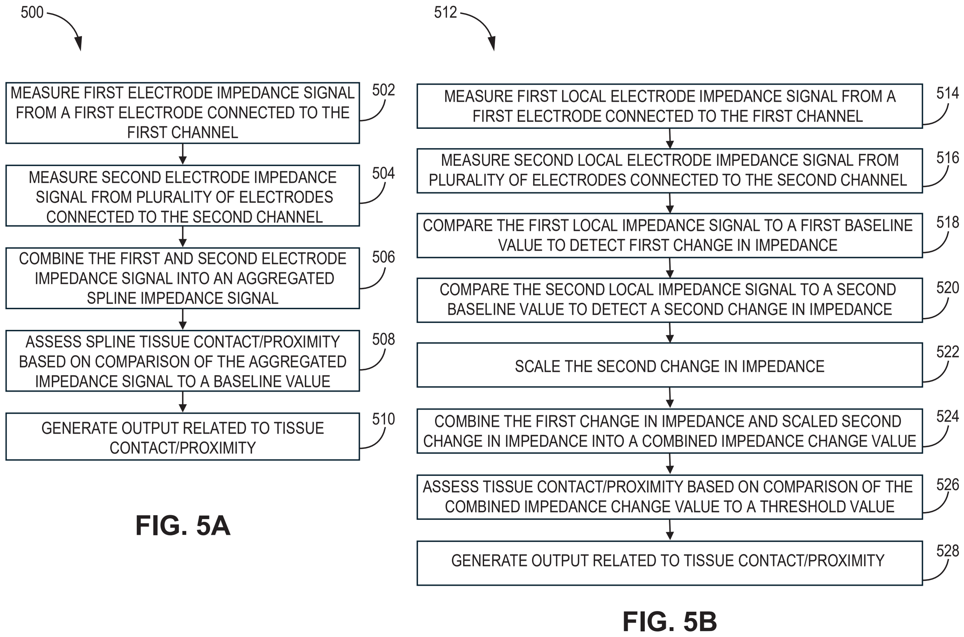

The electrode impedance signal 414 is measured in response to the constant current provided by the drive circuit 402. The voltage measured by each sense channel in combination with knowledge of the current provided by the drive circuit 402 allows an electrode impedance signal 414 to be determined. The electrode impedance signal is related to the impedance "seen" in the area adjacent to the electrode (or plurality of electrodes) and can be utilized to detect tissue contact/proximity as the impedance seen by the electrode decreases as the electrode comes into contact with tissue (this is due to the fact that tissue presents a higher impedance than the blood pool). As discussed in more detail below, the measured electrode impedance signal associated with the sense channel connected to a single electrode differs from the measured electrode impedance signal associated with the sense channel connected to a plurality of electrodes by virtue of the plurality of electrodes being connected in a parallel configuration. **FIGS. 4 and 5A**, **5B** describe in more detail how measured electrode impedance signals 414 associated with both sense channels connected to a single electrode and sense channels connected to a plurality of electrodes may be utilized to determine tissue contact/proximity.

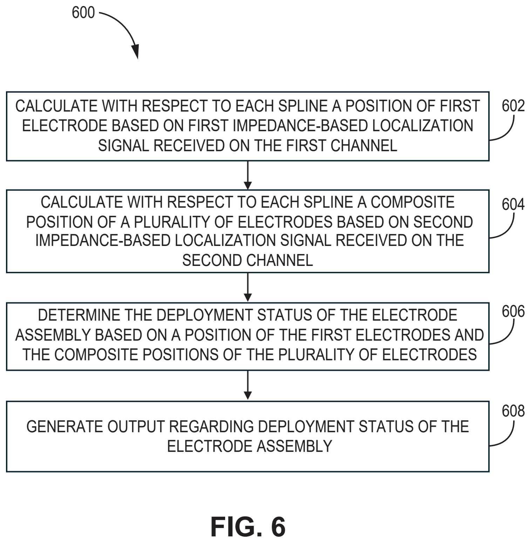

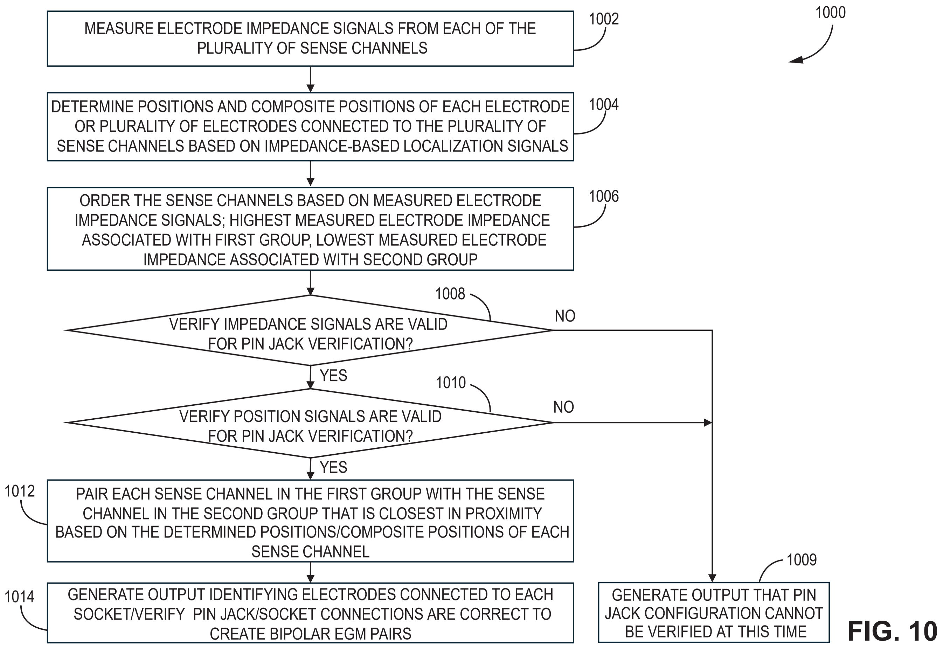

Impedance-based localization signals 418 refer to the measured signals utilized to locate the electrodes within the body. In some embodiments, localization and navigation system 130 (shown in **FIG. 1**) includes a plurality of surface patch pairs placed on the patient's skin. For example, a first pair of surface patch electrodes may include a front surface patch electrode and a back surface patch electrode, a second pair may include a right-side surface patch electrode and a left-side surface patch electrode, typically positioned on opposite sides of the body and creating axis that are orthogonal to one another. Localization and navigation system 130 applies voltage signals between the respective pairs of surface patch electrodes to generate an electric potential field (also referred to as an impedance field) across the patient's body, wherein the impedance-based localization signal 418 received or measured by each sense channel is utilized to locate the corresponding electrode or plurality of electrodes within the three-dimensional space of the patient's body. The impedance-based localization signal 418 received or measured by the second sense channel 400b and corresponding with the plurality of electrodes $304ai$, $304a_2$, and $304a_4$ will correspond with a point in space approximately between the plurality of electrodes (e.g., a "virtual" electrode located at the centroid of the physical electrodes $304ai$, $304a_2$, and $304a_4$). The impedance-based localization signal 418 associated with sense channels connected to a single electrode as well as impedance-based localization signals 418 associated with sense channels connected to a plurality of electrodes may be utilized - in addition to typical localization and navigation purposes - in operations related to shape detection (described with respect to **FIGS. 6-9**) and pin jack detection (described with respect to **FIG. 10**).

Electrogram (EGM) signals 416 are electrical cardiac signals measured from within the cardiac chamber. EGM signals 416 may be measured with respect to a single channel (e.g., unipolar signal, measured by either the first channel connected to a single electrode $304a_3$ or the second channel connected to a plurality of electrodes $304a_1$, $304a_2$, and $304a_4$) or a pair of channels (e.g., bipolar signal, measured between two channels, such as between the first channel and the second channel). In some applications, it is beneficial for a bipolar EGM signal to be based on signals measured with respect to electrodes located on the same spline. For example, a first EGM signal 416 may be based on signals by a first channel connected to the electrode $304a_3$ located on the first spline 302a and by a second channel connected to the electrodes $304a_1$, $304a_2$, and $304a_4$ also located on the first spline 302a. In some applications, the EGM pairs depend on the connection of the pin jacks associated with cable 111 (shown in **FIG. 1**) to the input sockets 110. A method of verifying the pin jacks of cable 111 are connected to the proper input sockets 110 to provide the desired EGM signals is described with respect to **FIG. 10** and utilizes both the electrode impedance signals 414 and impedance-based localization signals 418.

**FIG. 4** is a flowchart 420 illustrating steps implemented by the tissue contact module 122 (shown in **FIG. 1**) to assess spline tissue contact/proximity based on signals received from at least one sensing channel connected to a plurality of electrodes (e.g., second sensing channel 400b shown in **FIG. 3**). It should be noted, in some applications the term "contact status" is a binary determination, with the electrode either being "in contact" with the tissue or "not in contact" with the tissue. In other embodiments, the term "contact status" may include additional contact states, such as "intermittent contact". In still other embodiments, the term "contact status" may describe a proximity of the electrode to adjacent tissue. The term tissue contact/proximity is used throughout but may include any of these definitions.

Tissue contact/proximity based on measured electrode impedance for a single electrode is generally well understood. However, for sense channels connected to a plurality of electrodes the assessment changes. In general, the sensitivity of the measured electrode impedance signal connected to a plurality of electrodes is significantly less sensitive than the electrode impedance signal associated with a single electrode. Scaling the measured electrode impedance signal or redefining the threshold utilized to assess tissue contact/proximity may be utilized, but does not adequately address the issue which is that the impedance measured by the sense channel connected to a plurality of electrodes is always measured as a combined value, and therefore the metric derived is relevant to all of the connected electrodes, not just a single electrode. In the embodiment shown in FIG. 4, this problem is addressed by generating a value related to spline tissue contact/proximity, rather than individual electrode tissue contact/proximity.

At step 422, a first electrode impedance signal is received or measured from first electrode $304a_3$ connected to the first sense channel 400a. For the basket assembly shown in **FIGS. 2A, 2B,** this may include receiving or measuring electrode impedance signals with respect to each of the plurality of sense channels connected to a single electrode (e.g., electrodes $304a_3$, $304b_3$, $304c_3$, $304d_3$, and $304e_3$).

At step 424, a second electrode impedance signal is received or measured from a plurality of electrodes (e.g., electrodes $304ai$, $304a_2$, $304a_4$) connected to the second sense channel 400b. For the basket assembly shown in **FIGS. 2A, 2B**, this may include receiving or measuring electrode impedance signals with respect to each of the plurality of channels connected to a plurality of electrodes (e.g., electrodes $304a_1$, $304a_2$, $304a_4$, electrodes $304bi$, $304b_2$, $304b_4$, electrodes $304c_1$, $304c_2$, $304c_4$, electrodes $304d_1$, $304d_2$, $304d_4$, and electrodes $304e_1$, $304e_2$, $304e_4$).

At step 426, tissue contact/proximity is assessed based on the received or measured first and second electrode impedance signals. Because the second electrode impedance signals measured by the channels connected to a plurality of electrodes, the measured electrode impedance signal is lower as a result of the plurality of electrodes being connected in parallel. This difference in measured electrode impedance signals may be accounted for in several ways. In one embodiment, described in more detail in **FIG. 5A**, the first and second electrode impedance signals are combined, and the combined value is compared to a baseline or threshold value to assess tissue/contact proximity. In another embodiment utilized either alone or in conjunction with the first method, the first electrode impedance signal is compared to a first baseline/threshold value to determine a first change in impedance and the second electrode impedance signal is compared to a second baseline/threshold value to assess a second change in impedance. The first and second changes in impedance are then combined and utilized to assess tissue/contact proximity. The term baseline value may be defined as the expected or previously measured electrode impedance signal when the electrodes are not in contact with adjacent tissue (i.e., the lowest expected impedance value). The baseline value may be assigned empirically based on observed electrode impedance signals measured when the electrodes are not in contact with adjacent tissue or may be calculated based on expected values

With respect to the basket assembly shown in **FIGS. 2A, 2B**, the tissue contact/proximity assessments are made with respect to each spline. That is, if the first electrode impedance signal is made with respect to electrode $304a_3$ from the first spline 302a, then the second electrode impedance signal is made with respect to electrodes $304a_1$, $304a_2$, and $304a_4$ associated with the first spline 302a. Separate assessments of the other splines 302b, 302c, 302d, and 302e may be based on first and second electrode impedance signals measured with respect to electrodes associated with each spline.

**FIGS. 5A, 5B** are flowcharts illustrating steps implemented by the tissue contact module to assess tissue contact/proximity based on local electrode impedance signals received from at least one sensing channel connected to a plurality of electrodes (e.g., second sensing channel 400b shown in **FIG. 3**). The method described in **FIG. 5A** describes a method in which the impedance signals from respective sensing channels are combined prior to comparing to a threshold value to determine tissue contact/proximity, the method described in **FIG. 5B** describes a method in which the impedance signals from each channel are compared to separate threshold values and then subsequently combined.

In the embodiment shown in **FIG. 5A**, at steps 502 and 504, first and second electrode impedance signals are received or measured. The first electrode impedance signal is received or measured by first sense channel connected to a first electrode (e.g., electrode $304a_3$) and the second electrode impedance signal is received or measured by a second sense channel connected to a plurality of electrodes (e.g., electrodes 304ai, $304a_2$, and $304a_4$).

At step 506, the first and second electrode impedance signals are combined into an aggregated impedance signal. For example, in one embodiment, the first and second electrode impedance signals are combined according to the following equation:

$$Imp_{spline} = (Imp_1 + n * Imp_2)/s$$

where $Imp_{spline}$ is the impedance of the spline, $Imp_1$ is the first impedance single measured with respect to the individual electrode connected to the first channel, $Imp_2$ is the second impedance single measured with respect to the plurality of electrodes connected to the second channel, n is the number of electrodes connected to the second channel (e.g., three in the example shown in **FIG. 3**), and s is the total number of channels (e.g., two in the example described here). In this way, the value $Imp_{spline}$ provides a combined impedance value that can be utilized to assess tissue contact of the spline in general, rather than of particular electrodes located on the spline. In other examples, the first and second electrode impedance signals may be combined according to other equations.

At step 508, spline tissue contact/proximity is assessed based on a comparison of the aggregated impedance signal to a baseline or threshold value. As described above, a measured electrode impedance signal increases as the electrodes come into closer proximity/contact with adjacent tissue, albeit with less sensitivity for channels connected to a plurality of electrodes. In general, if the aggregated impedance signal is greater than the threshold value (or greater than the baseline by a given amount), then some level of tissue proximity/contact is detected. However, rather than generate an output related to a particular electrode regarding tissue contact/proximity, the tissue contact/proximity assessment is made at a spline level, wherein the spline includes the electrode connected to the first channel and the plurality of electrodes connected to the second channel.

The threshold utilized to detect spline tissue contact/proximity may vary depending on the application. For example, one application may initiate pulse field ablation (PFA) treatment in response to all electrodes on a spline being in full contact with tissue. In this example, the aggregated impedance signal is be compared to a threshold value that is significantly greater than the baseline value (e.g., 30% greater than the baseline value). In other applications, the threshold may be satisfied if at least one of the electrodes is in full contact with the adjacent tissue or more than one electrode is in partial contact (i.e., close proximity) to adjacent tissue. In this example, the aggregated impedance signal may be compared to a lower threshold value (e.g., 16% greater than the baseline value) In still other applications, it may be desirable to set a threshold that can be reached with only one of the plurality of electrodes associated with the second channel to be in contact with tissue or a plurality of electrodes are in partial contact with adjacent tissue. In this application, an even lower threshold may be set (e.g., 4.5% greater than the baseline value). In other applications, various other thresholds may be selected depending on the application.

At step 510, an output is generated displaying the assessed tissue contact/proximity of each of the plurality of splines. With respect to the electrode assembly 109 shown in **FIGS. 2A and 2B,** a tissue contact/proximity output is generated and displayed for each of the plurality of splines (e.g. each of the five splines 302a-302e). The display may include only a tissue contact/proximity output (e.g., in contact, partial contact, no contact) or may also include information regarding the aggregated impedance measurement and/or first and second impedances measured by each channel.

In the embodiment shown in **FIG. 5B**, at steps 514 and 516, first and second electrode impedance signals are received or measured. The first electrode impedance signal is received or measured by first channel connected to a first electrode (e.g., electrode $304a_3$) and the second electrode impedance signal is received or measured by a second channel connected to a plurality of electrodes (e.g., electrodes $304a_1$, $304a_2$, and $304a_4$).

At step 518, the first electrode impedance signal is compared to a first baseline value to detect a first change in impedance from the baseline. For example, in some embodiments if the baseline impedance of an electrode is 300 ohms ($\Omega$) and the first electrode impedance signal measured is 350 $\Omega$, the change or delta in the values is 50 $\Omega$.

At step 520, the second electrode impedance signal is compared to a second baseline value to detect a second change in impedance from the second baseline. The first and second baseline values may be different.

At step 522 the second change in impedance value is scaled to allow comparison with the first change in impedance. In one embodiment, the change in impedance is scaled by multiplying by the number of electrodes connected to the sense channel (e.g., three in the example shown in **FIG. 3**).

At step 524, the first change in impedance and scaled second change in impedance are combined into a combined change in impedance value (e.g., first change in impedance is added to the scaled second change in impedance). In other embodiments, rather than combining the first change in impedance with the scaled second change in impedance, each change in impedance is compared to a threshold value to determine tissue contact/proximity of each channel. For the sense channel connected to a single electrode this is straight-forward, but for the sense channel connected to a plurality of electrodes, the tissue contact/proximity assessment based on the comparison is not with respect to a particular electrode, but rather with respect to a plurality of electrodes. Hence, in some embodiments it is beneficial to combine the first change in impedance and the scaled second change in impedance into a combined impedance change value and utilize that value to assess tissue contact/proximity of the spline as a whole.

At step 526, tissue contact/proximity is assessed based on a comparison of the combined impedance change value to a threshold value. impedance signal to a baseline or threshold value. As described above, a measured electrode impedance signal increases as the electrodes come into closer proximity/-contact with adjacent tissue, albeit with less sensitivity for channels connected to a plurality of electrodes. In general, if the aggregated impedance signal is greater than the threshold value (or greater than the baseline by a given amount), then some level of tissue proximity/contact is detected. As described previously, various thresholds may be selected based on the type of tissue contact/proximity desired to detect.

At step 528, an output is generated displaying the assessed tissue contact/proximity of each of the plurality of splines. With respect to the electrode assembly 109 shown in **FIGS. 2A and 2B**, a tissue contact/-proximity output is generated and displayed for each of the plurality of splines (e.g. each of the five splines 302a-302e). The display may include only a tissue contact/proximity output (e.g., in contact, partial contact, no contact) or may also include information regarding the combined impedance change value and/or first and second impedances measured by each channel.

**FIG. 6** is a flowchart 600 illustrating steps implemented by the shape detection module to detect the shape of the distal end of the medical device based on impedance-based localization signals received from a plurality of first channels and second channels.

At step 602, the position of a first electrode is determined based on the first impedance-based localization signal received from the first channel. In the example shown in **FIGS. 2A-2B**, the position of the first electrode is determined with respect to each of the plurality of splines (i.e., impedance-based localization is measured for each of the electrodes $304a_3$-$304e_3$). As described above with respect to **FIG. 3**, impedance-based localization signals 418 are one of the types of signals that can be measured by the respective sense channels.

At step 604, a composite position of a plurality of electrodes connected to the second sense channel is determined based on a second impedance-based localization signal received from the second channel. In the example shown in **FIG. 3**, the composite position is based on the impedance-based localization signals 418 generated by the second sense channel 400b and is representative of a "virtual" electrode located approximately at the centroid of the physical electrodes $304a_1$, $304a_2$, and $304a_4$. With respect to the embodiment shown in **FIGS.**

**2A-2B,** composite positions would be generated for the plurality of electrodes associated with each of the plurality of splines (e.g., first composite position generated for electrodes $304ai$, $304a_2$, $304a_4$ located on the first spline 302a, second composite position generated for electrodes $304b_1$, $304b_2$, $304b_4$ located on the second spline 302b, and so on).

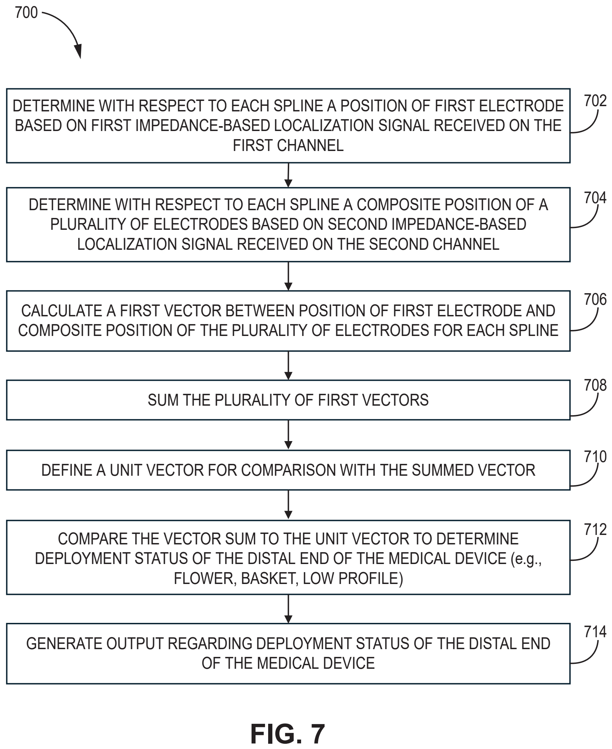

At step 606, the deployment status of electrode assembly 109 is determined based on the position of the first electrodes and the composite positions of the plurality of electrodes connected to sense channels. The determination is based on the relative position of the first electrodes to the composite positions of the plurality of electrodes. The method described with respect to **FIG. 7** describes one method of determining deployment status based on the respective position of the first electrodes and the composite position of the plurality of electrodes, but other methods may be utilized to efficiently determine the deployment status.

At step 608, an output is generated indicating the deployment status of the electrode assembly 109. In some embodiments, this may include indicating the deployment status as either "low profile", "basket", or "flower". In other embodiments, the output may graphically illustrate electrode assembly 109 in the determined deployment status.

**FIG. 7** is a flowchart 700 illustrating in additional detail steps implemented by the shape detection module to detect the shape of the distal end of the medical device based on impedance-based localization signals received from a plurality of first channels and second channels.

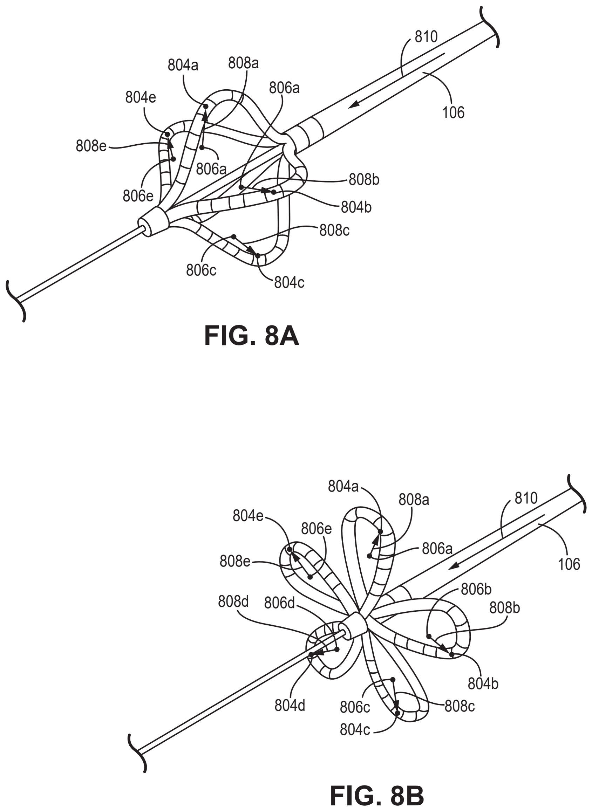

At step 702, the position of a first electrode is determined based on the first impedance-based localization signal received from the first channel. In the embodiment shown in **FIGS. 8A-8B,** the position of the first electrode with respect to each spline is illustrated by points 804a-804e.

At step 704, a composite position of a plurality of electrodes connected to the second sense channel is determined based on a second impedance-based localization signal received from the second channel. In the embodiment shown in **FIGS. 8A-8B,** the composite position is illustrated by points 806a-806e.

At step 706, a vector is calculated between the position of the first electrode and the composite position of the plurality of electrodes for each spline. In the embodiment shown in in **FIGS. 8A-8B**, the calculated vector is illustrated by arrows 808a-808e. As illustrated in the comparison between the vectors calculated when the electrode assembly 109 is deployed in the basket configuration (**FIG. 8A**) and the flower configuration (**FIG. 8B**). In the flower configuration, the calculated vectors 808a-808e are more orthogonal to the longitudinal axis 810 of the shaft 106 and point outward in all different directions. In the basket configuration, the calculated vectors 808a-808e are less orthogonal to the longitudinal axis 810 of the shaft 106 and - while still pointing generally outward - have a longitudinal component that is oriented in the same direction. Although not shown, when the electrode assembly 109 is deployed in the low-profile configuration the calculated vector 806a-806e will point in the same direction and approximately parallel to the longitudinal axis 810 of the shaft 106. In some embodiments, the length of the vectors 808a-808e are normalized so that each vector has the same length (e.g., a length of "1").

At steps 708-712, the calculated vectors 808a-808e are utilized to determine the deployment status. There are a number of ways of utilizing these vectors 808a-808e, with the steps illustrated in steps 708-712 being one example. At step 708, the plurality of vectors 808a-808e (or normalized vectors) are summed to generate a summed vector denoted "S". In some embodiments the summed vector S may also be normalized to generate a normalized summed vector "$NS$".

At step 710, a unit vector is defined to provide a vector that can be compared to the summed vector or normalized summed vector. The unit vector can be defined in a number of ways. In one embodiment, the unit vector is the unit normal vector of a plane defined to best fit the plurality of positions 804a-804e and 806a-806e found at steps 702 and 704 (although in some embodiments the unit normal vector could be defined with respect to a plane that best fits only electrode positions 804a-804e or only the composite electrode positions 806a-806e). The unit normal vector is defined as perpendicular (or normal) to the defined plane of best fit. In other embodiments, the vector to be compared to the summed vector or normalized summed vector is based on the orientation of the shaft 106 (e.g., vector defined along the longitudinal axis 810 of the shaft 106 based, for example, on magnetic sensors located in the shaft 106).

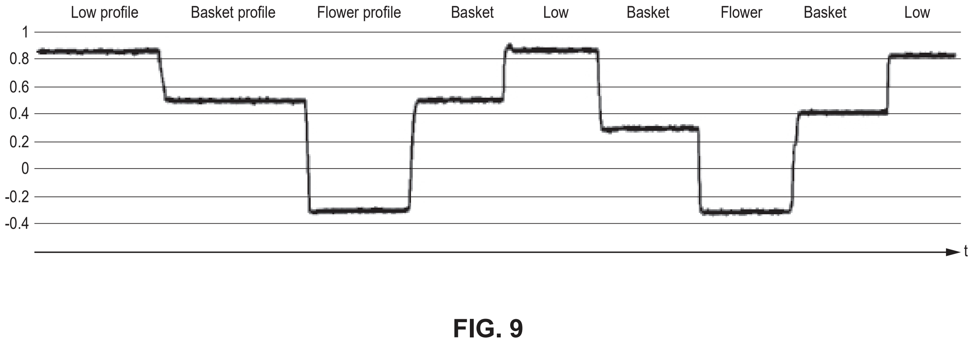

At step 712, the summed vector (or normalized summed vector) is compared to the unit vector defined at step 710. In some embodiments, the comparison is performed by performing a dot product between the summed vector and the unit vector defined at step 710. The results of the dot product operation for various configurations of the electrode assembly 109 (e.g., low profile, basket profile, and flower profile) is illustrated in the graph shown in **FIG. 9**. As illustrated, for low profile configurations in which the summed vector points in the same direction as the unit normal vector defined at step 710 (depending on how the vectors are defined), the dot product provides a value close to '1'. For basket profile configurations in which the summed vector points in somewhat the same direction as the unit normal vector defined at step 710, the dot product provides a value equal to about '0.5', and for flower profile configurations in which the summed vectors are perpendicular or maybe even at an angle greater than 90° to the unit normal vector defined at step 710, then the dot product is close to '0' or even slightly negative.

At step 714, an output is generated describing the deployment status of the electrode assembly. As described above, this may include indicating the deployment status as either "low profile", "basket", or "flower". In other embodiments, the output may graphically illustrate the electrode assembly 109 in the determined deployment status.

**FIG. 10** is a flowchart 1000 illustrating steps implemented by the pin jack detection module 126 (shown in **FIG. 1**) to ensure pin jacks associated with cable 111 are connected to the proper input sockets 110 (as shown in **FIG. 1**) to generate the desired bipolar electrogram (EGM) pairs. The method includes, generally, receiving inputs from each of the pin jacks connected to the input socket (i.e., each sense channel) without knowledge of which electrodes are necessarily associated with each sense channel. This is determined/verified by the steps illustrated in FIG. 10, such that at the end of the process the sense channels associated with select electrodes (e.g., the sense channels associated with electrodes on the same spline) are determined and can be utilized in subsequent operations as a bipolar pair (e.g., to form bipolar EGM signals based on signals received from two sense channels). In some embodiments, having determined the desired pin jack/input socket configuration, instructions can be provided to physically move the pin jacks to selected input sockets. In other embodiments, having determined the desired pin jack/input socket configuration, the desired pairs of pin jacks/input sockets can be selected in software. This embodiment is directed to the example illustrated in **FIGS. 2A, 2B, and 3**, in which each spline (e.g., spline 302a) includes a one electrode (e.g., electrode $304a_3$) connected to a first sense channel and three electrodes (e.g. electrodes $304a_1$, $304a_2$, and $304a_4$) connected to a second sense channel. In this example, it is desirable that these sense channels associated with electrodes on a single spline be organized or paired together to measure a bipolar EGM signal between the respective channels, rather than create bipolar pairs between electrodes located on different splines. In other embodiments, other pairings may be desired and the algorithm may be modified accordingly.

According to some aspects, prior to beginning the verification process at step 1002, a determination is made whether to initiate the verification process. For example, depending on the electrode array 109 being utilized, some configurations do not lend themselves to accurate measurements during some deployment configurations. For example, with the basket configuration illustrated in FIGS. 2A, 2B, when the electrode array 109 is deployed in the basket configuration (FIG. 2A) or flower configuration (FIG. 2B), the signals received from the electrodes are typically separate from one another and may be utilized to verify pin jack verification. However, if the electrode array 109 is in the low-profile configuration (not shown), then some of the electrodes may be in contact with one another, resulting in distorted measurements. In this example, the verification process would only be initiated in response to the electrode array 109 transitioning from the low-profile configuration to either the basket configuration or the flower configuration. In some embodiments, an output is generated to indicate that the verification process cannot be initiated and that therefore the pin jack configuration cannot be verified.

Assuming the verification process is initiated, then at step 1002 electrode impedance signals are received or measured with respect to each of the plurality of sense channels, including at least some sense channels connected to a single electrode and other sense channels connected to a plurality of electrodes. For example, in the embodiment shown in **FIGS. 2A, 2B, and 3,** five sense channels are connected to single electrodes (e.g., electrodes $304a_3$-$304e_3$) and five sense channels are connected to a plurality of electrodes. For example, one sense channel is connected to electrodes $304a_1$, $304a_2$, and $304a_4$, and a second sense channel is connected to electrodes $304b_1$, $304b_2$, and $304b_4$.

At step 1004, positions are determined based on impedance-based localization signals received on each channel. At this point, it is not yet verified which of the plurality of sense channels are connected to single electrodes and which of the plurality of sense channels are connected to a plurality of electrodes. Thus, at least some of the positions determined correspond with individual electrodes and at least some of the determined positions correspond with composite positions (i.e., "virtual" electrodes) associated with a plurality of electrodes connected to a single sense channel.

At step 1006, the sense channels are organized based on the electrode impedance signals received or measured at step 1002. In one example, the sense channels are ordered from highest electrode impedance measured to lowest electrode impedance measured. The assumption made is that the sense channels connected to a single electrode will exhibit a higher electrode impedance measurement. Based on knowledge regarding the total number of channels connected to single electrodes (e.g., five in the example illustrated in **FIGS. 2A, 2B, and 3**), the five highest electrode impedance measurements are assumed to be associated with sense channels connected to a single electrode and are organized into a first group. The remaining sense channels (i.e., the lowest electrode impedance measurements) are organized into a second group.

At step 1008, impedance signals are reviewed to determine if they are valid for use in pin jack verification. In some embodiments it would be beneficial to verify that each of the electrodes is located in the blood pool at the time of measurement and that the corresponding electrode impedance measurements vary based on the number of electrodes connected to each channel rather than on contact with adjacent tissue. This can be assessed in a number of ways, but in general the electrode impedance data should be grouped neatly into high impedance measurements associated with sense channels connected to a single electrode and low impedance measurements associated with sense channels connected to a plurality of electrodes. If the collected electrode impedance measurements cannot be neatly organized into two groups, this indicates that one or more of the electrodes may be in contact with adjacent tissue or may be in shorting to an adjacent electrode, making it difficult to distinguish which of the sense channels are connected to single electrodes and which of the sense channels are connected to a plurality of electrodes.

In one embodiment, after sorting the sense channels based on electrode impedance into two groups, the range of impedances associated with each group is calculated. That is, a first impedance range is calculated as the difference between the highest and lowest impedance in the first group and a second impedance range is calculated as the difference between the highest and lowest impedance in the second group. In addition, the spread between the two group is calculated (i.e., the minimum impedance value associated with the first group is subtracted from the maximum impedance value associated with the second group). In some embodiments, if the spread is compared to the maximum of the two ranges to determine if the electrode impedance data is valid for pin jack verification (in some embodiments the spread is compared to parameterized value generated based on the maximum of the two ranges). In general, if the spread is less than the parametrized value of the maximum of the two ranges this indicates the electrode impedance measurements are not organized into two distinct groups and that the electrode impedance measurements should be utilized to verify pin jack configuration.

If at step 1008 it is determined that the impedance signals cannot be utilized for pin jack verification, then at step 1009 an output is generated indicating to the user that the pin jack configuration cannot be verified at this time. Subsequently, the verification process could be restarted with new electrode impedance and position measurements. IF at step 1008 it is determined that the impedance signals can be utilized for pin jack verification, then the process continues at step 1010.

At step 1010, position signals are reviewed to determine if they are valid for use in pin jack verification. In general, it is assumed that the impedance-based location of an individual electrode connected to a first sense channel will be closest in proximity to the impedance-based composite position of the plurality of electrodes located on the same spline and associated with a second sense channel. However, bending or other physical deformations of the electrode array within the patient's body may make this assumption untrue. At step 1010, the determined positions/composite positions are utilized to determine whether deformations of the electrode array may make it difficult to use the positions/composite positions of each channel to pair channels associated with electrodes on the same spline with one another to create the desired bipolar EGMs. As with step 1008, a number of methods may be utilized to make this determination.

In one example, a first position calculated with respect to a first sense channel is selected and distances are calculated between this position and the positions/composite positions calculated for the other sense channels (e.g., in the example provided, nine distances would be calculated). The smallest distance (referred to herein as "D1") and the second smallest distance (referred to herein as "D2") are selected and compared to one another. In a best case scenario the smallest distance D1 represents the distance between the position an individual electrode located on a first spline (e.g., electrode $304a_3$) and the composite position of electrodes $304ai$, $304a_2$, and $304a_4$ located on the same spline. Likewise, in the best case scenario the second smallest distance D2 will represent the distance between the individual electrode on the first spline and a position or composite position of electrodes on different splines (and therefore much farther away). That is, the smallest distance D1 should be significantly smaller than the second smallest distance D2. This can be tested in a number of ways using thresholds or parameterized values. For example, the smallest distance D1 may be parameterized by multiplying the smallest distance D1 by a selected value and then comparing the parameterized value to the second smallest distance D2. If the second smallest distance D2 is less than the parameterized value, this indicates that the positions/composite positions of the electrodes make it difficult to rely on distance in determining if electrodes are located on the same spline. In this example, if the distances cannot be utilized to determine if the sense channels are connected to electrodes on the same spline, then at step 1009, an output is generated that indicates that the pin jack configuration cannot be verified at this time. If the second smallest distance D2 is not less than the parameterized value, this indicates that the position of the electrodes connected to the respective sense channels can be utilized to verify the sense channels connected to electrodes on the same spline. The same operation would be repeated for each sense channel. If at step 1010 it is determined that the positions/composite positions associated with each of the sense channels are located relative to one another in such a way that electrodes on the same spline can be identified, then the process continues at step 1012.

At step 1012, each sense channel in the first group is paired with a sense channel from the second group that is located closest in proximity to the electrode associated with the sense channel in the first group based on the determined positions/composite positions of the electrodes associated with each sense channel. For example, assume a selected sense channel from the first group corresponds with electrode $304a_3$. The impedance-based location of electrode $304a_3$ (known from step 1004) is compared to the composite positions associated with each of the sense channels in the second group (which corresponds with each of the sense channels connected to a plurality of electrodes). The composite position from the second group that is located closest in proximity to the position of electrode $304a_3$ can be determined to be associated with the sense channel connected to electrodes $304ai$, $304a_2$, and $304a_4$. In this way, pairs are created between a sense channel selected from the first group and a sense channel selected from the second group. The selected sense channel pairs should correspond with electrodes located on the same spline and can therefore be utilized to measure bipolar EGM pairs based on EGM signals measured by each channel. This process is repeated until each of the sense channels in the first group has been paired with a sense channel in the second group. This example assumes that the number of sense channels associated with a single electrode is equal to the number of sense channels associated with a plurality of electrodes. The resulting pairs can be used to create the desired pairings between pin jacks and input sockets, either physically or in software, or to verify that the connections are correct.

At step 1010, an output is generated identifying the electrodes connected to each socket and verifying that the desired pairings of sense channels are connected properly to generate the desired bipolar EGM pairs.

While the invention has been described with reference to an exemplary embodiment(s), it will be understood by those skilled in the art that various changes may be made and equivalents may be substituted for elements thereof without departing from the scope of the invention. In addition, many modifications may be made to adapt a particular situation or material to the teachings of the invention without departing from the essential scope thereof. Therefore, it is intended that the invention not be limited to the particular embodiment(s) disclosed, but that the invention will include all embodiments falling within the scope of the appended claims.

Clause 1. A computer-implemented method of assessing tissue contact for a plurality of electrodes located on a medical device, the method comprising: measuring, using a first sense channel, a first electrode impedance signal associated with a first electrode; measuring, using a second sense channel, a second electrode impedance signal associated with at least a second and third electrode, wherein the second and third electrodes are electrically connected to each other but not to the first electrode; and assessing tissue contact/proximity based on the first electrode impedance signal and the second electrode impedance signal.

Clause 2. The computer-implemented method of clause 1, wherein assessing tissue contact/proximity further includes: combining the first electrode impedance signal and the second electrode impedance signal into an aggregated electrode impedance signal; and comparing the aggregated electrode impedance signal to a baseline value to determine tissue contact/proximity.

Clause 3. The computer-implemented method of clause 2, wherein comparing the aggregated electrode impedance signal to a baseline value includes comparing the aggregated electrode impedance signal to a threshold value, wherein tissue contact is detected if the aggregated electrode impedance signal is greater than the threshold value.

Clause 4. The computer-implemented method of clause 3, wherein the threshold value is selected as representative of both the first electrode and at least one of the second and third electrodes being in contact with tissue.

Clause 5. The computer-implemented method of clause 3, wherein the threshold value is selected as representative of at least one of the first electrode or the second and third electrodes being in contact with tissue.

Clause 6. The computer-implemented method of any of clauses 2 to 5, wherein combining the first electrode impedance signal and the second electrode impedance signal into an aggregated electrode impedance signal includes scaling the second electrode impedance signal to a value representative of a single electrode impedance signal.

Clause 7. The computer-implemented method of clause 6, wherein scaling the second electrode impedance signal includes multiplying the second electrode impedance signal by a number of electrodes connected to the second sense channel.

Clause 8. The computer-implemented method of clause 7, wherein combining the first electrode impedance signal and the second electrode impedance signal further includes adding the first electrode impedance signal to the scaled second electrode impedance signal and dividing by a number of sense channels.

Clause 9. The computer-implemented method of any of clauses 1 to 8, wherein assessing tissue contact/proximity further includes: comparing the first electrode impedance signal to a first baseline value to detect a first change in impedance; comparing the second electrode impedance signal to a second baseline value to detect a second change in impedance; scaling the second change in impedance to a value representative of single electrode impedance measurements; combining the first change in impedance and the scaled second change in impedance; and assessing tissue contact/proximity based on a comparison of the combined change in impedance to a threshold value.

Clause 10. The computer-implemented method of clause 9, wherein scaling the second change in impedance includes multiplying the second change in impedance by a number of electrodes connected to the second sense channel.

Clause 11. The computer-implemented method of any of clauses 1 to 10, wherein the first, second and third electrodes are located on a single spline.

Clause 12. The computer-implemented method of clause 11, further including generating an output indicating tissue contact/proximity of the spline based on the measured first electrode impedance signal and the measured second electrode impedance signal.

Clause 13. A computer-readable medium storing instructions that, when executed by a processor, cause the processor to perform a method of assessing tissue contact for a plurality of electrodes located on a medical device, the method comprising:

receiving, via a first sense channel, a first electrode impedance signal associated with a first electrode (step 402, 502, 514);
receiving, via a second sense channel, a second electrode impedance signal associated with at least a second and third electrode, wherein the second and third electrodes are electrically connected to each other but not to the first electrode (step 404, 504, 516); and
assessing tissue contact/proximity based on the first electrode impedance signal and the second electrode impedance signal (step 406).

Clause 14. The computer-readable medium of clause 13, wherein assessing tissue contact/proximity further includes:

combining the first electrode impedance signal and the second electrode impedance signal into an aggregated electrode impedance signal (step 506); and
comparing the aggregated electrode impedance signal to a baseline value to determine tissue contact/proximity (step 508).

Clause 15. The computer-readable medium of clause 13, wherein assessing tissue contact/proximity further includes:

comparing the first electrode impedance signal to a first baseline value to detect a first change in impedance (step 518);
comparing the second electrode impedance signal to a second baseline value to detect a second change in impedance (step 520);
scaling the second change in impedance to a value representative of single electrode impedance measurements (step 522);
combining the first change in impedance and the scaled second change in impedance (step 524); and
assessing tissue contact/proximity based on a comparison of the combined change in impedance to a threshold value (step 526).

Clause 16. A system (100) for assessing tissue contact for a plurality of electrodes (304a) located on a medical device (102), the system comprising:
a tissue contact module (122) configured to:

measure, using a first sense channel (400a), a first electrode impedance signal (414) associated with a first electrode (304a1);
measure, using a second sense channel (400b), a second electrode impedance signal (414) associated with at least a second and third electrode (304a2, 304a3), wherein the second and third electrodes (304a2, 304a3) are electrically connected to each other but not to the first electrode (304a1); and assess tissue contact/proximity based on the first electrode impedance signal (414) and the second electrode impedance signal (414).

Clause 17. The system of clause 16, wherein the tissue contact module (122) is configured to assess tissue contact/proximity by:

combining the first electrode impedance signal (414) and the second electrode impedance signal (414) into an aggregated electrode impedance signal; and comparing the aggregated electrode impedance signal to a baseline value to determine tissue contact/-proximity.

Clause 18. The system of clause 17, wherein comparing the aggregated electrode impedance signal to a baseline value includes comparing the aggregated electrode impedance signal to a threshold value, wherein tissue contact is detected if the aggregated electrode impedance signal is greater than the threshold value, optionally (i) wherein the threshold value is selected as representative of both the first electrode and at least one of the second and third electrodes being in contact with tissue, or (ii) wherein the threshold value is selected as representative of at least one of the first electrode or the second and third electrodes being in contact with tissue.

Clause 19. The system of clause 17 or 18, wherein combining the first electrode impedance signal (414) and the second electrode impedance signal (414) into an aggregated electrode impedance signal includes scaling the second electrode impedance signal to a value representative of a single electrode impedance signal, optionally wherein scaling the second electrode impedance signal (414) includes multiplying the second electrode impedance signal by a number of electrodes connected to the second sense channel, optionally wherein combining the first electrode impedance signal (414) and the second electrode impedance signal (414) further includes adding the first electrode impedance signal (414) to the scaled second electrode impedance signal and dividing by a number of sense channels.

Clause 20. The system of any of clauses 16 to 19, wherein assessing tissue contact/proximity further includes:

comparing the first electrode impedance signal (414) to a first baseline value to detect a first change in impedance; comparing the second electrode impedance signal to a second baseline value to detect a second change in impedance; scaling the second change in impedance to a value representative of single electrode impedance measurements; combining the first change in impedance and the scaled second change in impedance; and assessing tissue contact/proximity based on a comparison of the combined change in impedance to a threshold value, optionally wherein scaling the second change in impedance includes multiplying the second change in impedance by a number of electrodes connected to the second sense channel.

Clause 21. The system of any of clauses 16 to 20, wherein the first, second and third electrodes are located on a single spline (302), optionally further including generating an output indicating tissue contact/proximity of the spline (302) based on the measured first electrode impedance signal (414) and the measured second electrode impedance signal (414).

Clause 22. A computer-implemented method of determining a deployment status of an electrode assembly located at a distal end of a catheter having a plurality of splines and a plurality of electrodes on each spline, the method comprising:

calculating with respect to each spline a position of a first electrode located on each spline based on a first impedance-based localization signal received on a first sense channel (step 602, 702); calculating with respect to each spline a composite position of at least second and third electrodes located on each spline based on a second impedance-based localization signal received on a second sense channel (step 604, 704); and

determining a deployment status of the electrode assembly based on the position of the first electrode and the composite position of the at least second and third electrodes (step 606).

Clause 23 The computer-implemented method of clause 22, wherein the deployment status of the electrode assembly includes a low profile status, a basket profile status, and a flower profile status.

Clause 24. The computer-implemented method of clauses 22 or 23, wherein determining the deployment status of the electrode assembly further includes:

calculating, for each spline, vectors between the position of the first electrode and the composite position of at least the second and third electrodes for each spline (step 706); and determining a deployment status based on the vectors calculated for each of the plurality of splines (step 712).

Clause 25. The computer-implemented method of clause 24, further including:

summing the vectors calculated for each of the plurality of splines to generate a summed vector (step 708); comparing the summed vector to a unit vector (step 710); and

determining deployment status of the electrode assembly based on the comparison of the summed vector to the unit vector (step 712).

Clause 26. The computer-implemented method of clause 25, wherein the unit vector is a unit normal vector of a plane defined to best fit the position of the first electrodes associated with each spline, a plane defined to best fit the composition position of the second and third electrodes associated with each spline, or a plane defined to best fit both the position of the first electrodes and the composite position of the second and third electrodes associated with each spline.

Clause 27. The computer-implemented method of clauses 25 or 26, wherein the summed vector is compared to the unit vector using a dot product function, wherein the dot product output is utilized to determine the deployment status of the distal end of the catheter.

Clause 28. The computer-implemented method of clauses 25, 26 or 27, further including:

calculating second vectors between the position of the first electrodes on adjacent splines; and
determining a deployment status based on the first vectors calculated for each of the plurality of splines and the second vectors between the position of the first electrodes on adjacent splines.

Clause 29. A computer-implemented method of confirming proper pin jack connection order for connection to a plurality of electrodes located at a distal end of a medical device, the method comprising:

measuring electrode impedance signals associated with each of a plurality of sense channels (step 1002);
determining a position/composite position associated with each of the plurality of sense channels based on impedance-based localization signals measured by each sense channel, the position/composite positions corresponding with electrodes or groups of electrodes connected to each of the plurality of sense channels (step 1004);
organizing the plurality of sense channels into a first group of sense channels and a second group of sense channels based on the measured electrode impedance signals (step 1006);
pairing each sense channel in the first group with the sense channel in the second group that is closest in proximity based on the determined positions/composite positions of each of the plurality of sense channels (step 1012); and
measuring bipolar electrogram (EGM) signals based on the pairing of each sense channel in the first group with a sense channel in the second group (step 1014).

Clause 30. The computer-implemented method of clause 29, wherein at least some of the sense channels are connected to single electrodes located on an electrode assembly located at the distal end of a medical device and at least some of the sense channels are connected to a plurality of electrodes located on the electrode assembly located at the distal end of the medical device.

Clause 31. The computer-implemented method of clauses 29 or 30, wherein the measured electrode impedance signals associated with sense channels connected to single electrodes are greater than the measured electrode impedance signals associated with sense channels connected to a plurality of electrodes.

Clause 32. The computer-implemented method of clauses 29, 30 or 31, wherein the electrode assembly located at the distal end of the medical device is comprised of a plurality of splines, each spline having an plurality of electrodes, wherein at least some of the sense channels are connected to a single electrode on each spline and at least some of the channels are connected to a plurality of electrodes on the same spline.

Clause 33. A computer-readable medium storing instructions that, when executed by a processor, cause the processor to perform a method of assessing tissue contact for a plurality of electrodes located on a medical device, the method comprising:

measuring, using a first sense channel, a first electrode impedance signal associated with a first electrode (step 402, 502, 514);
measuring, using a second sense channel, a second electrode impedance signal associated with at least a second and third electrode, wherein the second and third electrodes are electrically connected to each other but not to the first electrode (step 404, 504, 516); and
assessing tissue contact/proximity based on the first electrode impedance signal and the second electrode impedance signal (step 406).

Clause 34. A computer-readable medium storing instructions that, when executed by a processor, cause the processor to perform a method of determining a deployment status of an electrode assembly located at a distal end of a catheter having a plurality of splines and a plurality of electrodes on each spline, the method comprising:

calculating with respect to each spline a position of a first electrode located on each spline based on a first impedance-based localization signal received on a first sense channel (step 602, 702);
calculating with respect to each spline a composite position of at least second and third electrodes located on each spline based on a second impedance-based localization signal received on a second sense channel (step 604, 704); and

determining a deployment status of the electrode assembly based on the position of the first electrode and the composite position of the at least second and third electrodes (step 606).

Clause 35. A computer-readable medium storing instructions that, when executed by a processor, cause the processor to perform a method of confirming proper pin jack connection order for connection to a plurality of electrodes located at a distal end of a medical device, the method comprising:

measuring electrode impedance signals associated with each of a plurality of sense channels (step 1002);
determining a position/composite position associated with each of the plurality of sense channels based on impedance-based localization signals measured by each sense channel, the position/composite positions corresponding with electrodes or groups of electrodes connected to each of the plurality of sense channels (step 1004);
organizing the plurality of sense channels into a first group of sense channels and a second group of sense channels based on the measured electrode impedance signals (step 1006);
pairing each sense channel in the first group with the sense channel in the second group that is closest in proximity based on the determined positions/composite positions of each of the plurality of sense channels (step 1012); and
measuring bipolar electrogram (EGM) signals based on the pairing of each sense channel in the first group with a sense channel in the second group (step 1014).

**Claims**

1. A computer-implemented method of assessing tissue contact for a plurality of electrodes located on a medical device, the method comprising:

receiving, via a first sense channel, a first electrode impedance signal associated with a first electrode (step 402, 502, 514);
receiving, via a second sense channel, a second electrode impedance signal associated with at least a second and third electrode, wherein the second and third electrodes are electrically connected to each other but not to the first electrode (step 404, 504, 516); and
assessing tissue contact/proximity based on the first electrode impedance signal and the second electrode impedance signal (step 406).

2. The computer-implemented method of claim 1, wherein assessing tissue contact/proximity further includes:

combining the first electrode impedance signal and the second electrode impedance signal into an aggregated electrode impedance signal (step 506); and
comparing the aggregated electrode impedance signal to a baseline value to determine tissue contact/proximity (step 508).

3. The computer-implemented method of claim 2, wherein comparing the aggregated electrode impedance signal to a baseline value includes comparing the aggregated electrode impedance signal to a threshold value, wherein tissue contact is detected if the aggregated electrode impedance signal is greater than the threshold value, optionally (i) wherein the threshold value is selected as representative of both the first electrode and at least one of the second and third electrodes being in contact with tissue, or (ii) wherein the threshold value is selected as representative of at least one of the first electrode or the second and third electrodes being in contact with tissue.

4. The computer-implemented method of claims 2 or 3, wherein combining the first electrode impedance signal and the second electrode impedance signal into an aggregated electrode impedance signal includes scaling the second electrode impedance signal to a value representative of a single electrode impedance signal, optionally wherein scaling the second electrode impedance signal includes multiplying the second electrode impedance signal by a number of electrodes connected to the second sense channel, optionally wherein combining the first electrode impedance signal and the second electrode impedance signal further includes adding the first electrode impedance signal to the scaled second electrode impedance signal and dividing by a number of sense channels.

5. The computer-implemented method of any of claims 1 to 4, wherein assessing tissue contact/proximity further includes:

comparing the first electrode impedance signal to a first baseline value to detect a first change in impedance (step 518);
comparing the second electrode impedance signal to a second baseline value to detect a second change in impedance (step 520);
scaling the second change in impedance to a value representative of single electrode impedance measurements (step 522);
combining the first change in impedance and the scaled second change in impedance (step 524); and

assessing tissue contact/proximity based on a comparison of the combined change in impedance to a threshold value (step 526), optionally wherein scaling the second change in impedance includes multiplying the second change in impedance by a number of electrodes connected to the second sense channel.

6. The computer-implemented method of any previous claim, wherein the first, second and third electrodes are located on a single spline, optionally further including generating an output indicating tissue contact/proximity of the spline based on the measured first electrode impedance signal and the measured second electrode impedance signal.

7. A computer-readable medium storing instructions that, when executed by a processor, cause the processor to perform a method of assessing tissue contact for a plurality of electrodes located on a medical device, the method comprising:

    receiving, via a first sense channel, a first electrode impedance signal associated with a first electrode (step 402, 502, 514);
    receiving, via a second sense channel, a second electrode impedance signal associated with at least a second and third electrode, wherein the second and third electrodes are electrically connected to each other but not to the first electrode (step 404, 504, 516); and
    assessing tissue contact/proximity based on the first electrode impedance signal and the second electrode impedance signal (step 406).

8. The computer-readable medium of claim 7, wherein assessing tissue contact/proximity further includes:

    combining the first electrode impedance signal and the second electrode impedance signal into an aggregated electrode impedance signal (step 506); and
    comparing the aggregated electrode impedance signal to a baseline value to determine tissue contact/proximity (step 508).

9. The computer-readable medium of claim 7, wherein assessing tissue contact/proximity further includes:

    comparing the first electrode impedance signal to a first baseline value to detect a first change in impedance (step 518);
    comparing the second electrode impedance signal to a second baseline value to detect a second change in impedance (step 520);
    scaling the second change in impedance to a value representative of single electrode impedance measurements (step 522);

combining the first change in impedance and the scaled second change in impedance (step 524); and
assessing tissue contact/proximity based on a comparison of the combined change in impedance to a threshold value (step 526).

10. A system (100) for assessing tissue contact for a plurality of electrodes (304a) located on a medical device (102), the system comprising:
a tissue contact module (122) configured to:

    measure, using a first sense channel (400a), a first electrode impedance signal (414) associated with a first electrode (304a1);
    measure, using a second sense channel (400b), a second electrode impedance signal (414) associated with at least a second and third electrode (304a2, 304a3), wherein the second and third electrodes (304a2, 304a3) are electrically connected to each other but not to the first electrode (304a1); and
    assess tissue contact/proximity based on the first electrode impedance signal (414) and the second electrode impedance signal (414).

11. The system of claim 10, wherein the tissue contact module (122) is configured to assess tissue contact/proximity by:

    combining the first electrode impedance signal (414) and the second electrode impedance signal (414) into an aggregated electrode impedance signal; and
    comparing the aggregated electrode impedance signal to a baseline value to determine tissue contact/proximity.

12. The system of claim 11, wherein comparing the aggregated electrode impedance signal to a baseline value includes comparing the aggregated electrode impedance signal to a threshold value, wherein tissue contact is detected if the aggregated electrode impedance signal is greater than the threshold value, optionally (i) wherein the threshold value is selected as representative of both the first electrode and at least one of the second and third electrodes being in contact with tissue, or (ii) wherein the threshold value is selected as representative of at least one of the first electrode or the second and third electrodes being in contact with tissue.

13. The system of claim 11 or 12, wherein combining the first electrode impedance signal (414) and the second electrode impedance signal (414) into an aggregated electrode impedance signal includes scaling the second electrode impedance signal to a value representative of a single electrode impedance signal, optionally wherein scaling the second electrode impedance signal (414) includes multiplying the second electrode impedance signal by a number of electrodes connected to the second sense channel, optionally wherein combining the first electrode impedance signal (414) and the second electrode impedance signal (414) further includes adding the first electrode impedance signal (414) to the scaled second electrode impedance signal and dividing by a number of sense channels.

14. The system of any of claims 10 to 13, wherein assessing tissue contact/proximity further includes:

> comparing the first electrode impedance signal (414) to a first baseline value to detect a first change in impedance;
> comparing the second electrode impedance signal to a second baseline value to detect a second change in impedance;
> scaling the second change in impedance to a value representative of single electrode impedance measurements;
> combining the first change in impedance and the scaled second change in impedance; and
> assessing tissue contact/proximity based on a comparison of the combined change in impedance to a threshold value, optionally wherein scaling the second change in impedance includes multiplying the second change in impedance by a number of electrodes connected to the second sense channel.

15. The system of any of claims 10 to 14, wherein the first, second and third electrodes are located on a single spline (302), optionally further including generating an output indicating tissue contact/proximity of the spline (302) based on the measured first electrode impedance signal (414) and the measured second electrode impedance signal (414).

**FIG. 1**

**FIG. 2A**

**FIG. 2B**

**FIG. 3**

FIG. 4

**FIG. 5A**

500

| MEASURE FIRST ELECTRODE IMPEDANCE SIGNAL FROM A FIRST ELECTRODE CONNECTED TO THE FIRST CHANNEL | 502 |

| MEASURE SECOND ELECTRODE IMPEDANCE SIGNAL FROM PLURALITY OF ELECTRODES CONNECTED TO THE SECOND CHANNEL | 504 |

| COMBINE THE FIRST AND SECOND ELECTRODE IMPEDANCE SIGNAL INTO AN AGGREGATED SPLINE IMPEDANCE SIGNAL | 506 |

| ASSESS SPLINE TISSUE CONTACT/PROXIMITY BASED ON COMPARISON OF THE AGGREGATED IMPEDANCE SIGNAL TO A BASELINE VALUE | 508 |

| GENERATE OUTPUT RELATED TO TISSUE CONTACT/PROXIMITY | 510 |

**FIG. 5B**

512

| MEASURE FIRST LOCAL ELECTRODE IMPEDANCE SIGNAL FROM A FIRST ELECTRODE CONNECTED TO THE FIRST CHANNEL | 514 |

| MEASURE SECOND LOCAL ELECTRODE IMPEDANCE SIGNAL FROM PLURALITY OF ELECTRODES CONNECTED TO THE SECOND CHANNEL | 516 |

| COMPARE THE FIRST LOCAL IMPEDANCE SIGNAL TO A FIRST BASELINE VALUE TO DETECT FIRST CHANGE IN IMPEDANCE | 518 |

| COMPARE THE SECOND LOCAL IMPEDANCE SIGNAL TO A SECOND BASELINE VALUE TO DETECT A SECOND CHANGE IN IMPEDANCE | 520 |

| SCALE THE SECOND CHANGE IN IMPEDANCE | 522 |

| COMBINE THE FIRST CHANGE IN IMPEDANCE AND SCALED SECOND CHANGE IN IMPEDANCE INTO A COMBINED IMPEDANCE CHANGE VALUE | 524 |

| ASSESS TISSUE CONTACT/PROXIMITY BASED ON COMPARISON OF THE COMBINED IMPEDANCE CHANGE VALUE TO A THRESHOLD VALUE | 526 |

| GENERATE OUTPUT RELATED TO TISSUE CONTACT/PROXIMITY | 528 |

600

CALCULATE WITH RESPECT TO EACH SPLINE A POSITION OF FIRST ELECTRODE BASED ON FIRST IMPEDANCE-BASED LOCALIZATION SIGNAL RECEIVED ON THE FIRST CHANNEL — 602

CALCULATE WITH RESPECT TO EACH SPLINE A COMPOSITE POSITION OF A PLURALITY OF ELECTRODES BASED ON SECOND IMPEDANCE-BASED LOCALIZATION SIGNAL RECEIVED ON THE SECOND CHANNEL — 604

DETERMINE THE DEPLOYMENT STATUS OF THE ELECTRODE ASSEMBLY BASED ON A POSITION OF THE FIRST ELECTRODES AND THE COMPOSITE POSITIONS OF THE PLURALITY OF ELECTRODES — 606

GENERATE OUTPUT REGARDING DEPLOYMENT STATUS OF THE ELECTRODE ASSEMBLY — 608

# FIG. 6

700

DETERMINE WITH RESPECT TO EACH SPLINE A POSITION OF FIRST ELECTRODE BASED ON FIRST IMPEDANCE-BASED LOCALIZATION SIGNAL RECEIVED ON THE FIRST CHANNEL — 702

DETERMINE WITH RESPECT TO EACH SPLINE A COMPOSITE POSITION OF A PLURALITY OF ELECTRODES BASED ON SECOND IMPEDANCE-BASED LOCALIZATION SIGNAL RECEIVED ON THE SECOND CHANNEL — 704

CALCULATE A FIRST VECTOR BETWEEN POSITION OF FIRST ELECTRODE AND COMPOSITE POSITION OF THE PLURALITY OF ELECTRODES FOR EACH SPLINE — 706

SUM THE PLURALITY OF FIRST VECTORS — 708

DEFINE A UNIT VECTOR FOR COMPARISON WITH THE SUMMED VECTOR — 710

COMPARE THE VECTOR SUM TO THE UNIT VECTOR TO DETERMINE DEPLOYMENT STATUS OF THE DISTAL END OF THE MEDICAL DEVICE (e.g., FLOWER, BASKET, LOW PROFILE) — 712

GENERATE OUTPUT REGARDING DEPLOYMENT STATUS OF THE DISTAL END OF THE MEDICAL DEVICE — 714

**FIG. 7**

**FIG. 8A**

**FIG. 8B**

FIG. 9

```
                                                          ┌─ 1000
┌──────────────────────────────────────────────┐  ┌ 1002
│ MEASURE ELECTRODE IMPEDANCE SIGNALS FROM EACH  │
│ OF THE PLURALITY OF SENSE CHANNELS             │
└──────────────────────────────────────────────┘
                        │
                        ▼
┌──────────────────────────────────────────────┐  ┌ 1004
│ DETERMINE POSITIONS AND COMPOSITE POSITIONS OF │
│ EACH ELECTRODE OR PLURALITY OF ELECTRODES      │
│ CONNECTED TO THE PLURALITY OF SENSE CHANNELS   │
│ BASED ON IMPEDANCE-BASED LOCALIZATION SIGNALS  │
└──────────────────────────────────────────────┘
                        │
                        ▼
┌──────────────────────────────────────────────┐  ┌ 1006
│ ORDER THE SENSE CHANNELS BASED ON MEASURED     │
│ ELECTRODE IMPEDANCE SIGNALS; HIGHEST MEASURED  │
│ ELECTRODE IMPEDANCE ASSOCIATED WITH FIRST      │
│ GROUP, LOWEST MEASURED ELECTRODE IMPEDANCE     │
│ ASSOCIATED WITH SECOND GROUP                   │
└──────────────────────────────────────────────┘
                        │
                        ▼
              ◇ 1008
        VERIFY IMPEDANCE SIGNALS ARE VALID    ── NO ──┐
        FOR PIN JACK VERIFICATION?                    │
                        │ YES                          │
                        ▼                              │
              ◇ 1010                                   │
        VERIFY POSITION SIGNALS ARE VALID    ── NO ────┤
        FOR PIN JACK VERIFICATION?                     │
                        │ YES                          │
                        ▼                              │
┌──────────────────────────────────────────────┐ 1012 │
│ PAIR EACH SENSE CHANNEL IN THE FIRST GROUP     │      │
│ WITH THE SENSE CHANNEL IN THE SECOND GROUP     │      │
│ THAT IS CLOSEST IN PROXIMITY BASED ON THE      │      │
│ DETERMINED POSITIONS/COMPOSITE POSITIONS OF    │      │
│ EACH SENSE CHANNEL                             │      │
└──────────────────────────────────────────────┘      │
                        │                              ▼
                        ▼                     ┌──────── 1009 ────────┐
┌────────────────────────────────────────┐1014│ GENERATE OUTPUT THAT │
│ GENERATE OUTPUT IDENTIFYING ELECTRODES   │    │ PIN JACK CONFIGURATION│
│ CONNECTED TO EACH SOCKET/VERIFY PIN      │    │ CANNOT BE VERIFIED    │
│ JACK/SOCKET CONNECTIONS ARE CORRECT TO   │    │ AT THIS TIME          │
│ CREATE BIPOLAR EGM PAIRS                 │    └──────────────────────┘
└────────────────────────────────────────┘
```

**FIG. 10**

| | Europäisches Patentamt European Patent Office Office européen des brevets | **EUROPEAN SEARCH REPORT** | Application Number EP 26 15 1413 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/233235 A1 (EBERT HENNING [DE] ET AL) 28 July 2022 (2022-07-28) | 1-3,6-8, 10-12,15 | INV. A61B5/0538 |
| A | * paragraphs [0023], [0037] - [0038] * <br> * figure 1 * | 4,5,9, 13,14 | A61B5/287 <br> A61B5/06 <br> A61B5/00 |
| | ----- | | |
| X | US 2023/112251 A1 (GOVARI ASSAF [IL] ET AL) 13 April 2023 (2023-04-13) | 1-3,6-8, 10-12 | |
| A | * paragraphs [0050] - [0054] * <br> * figures 3b, 4 * | 4,5,9, 13,14 | |
| | ----- | | |
| X | US 2024/325066 A1 (SYRKIN-NIKOLAU JUDY [US] ET AL) 3 October 2024 (2024-10-03) | 1-3,7,8, 10-12 | |
| A | * paragraphs [0148] - [0151] * | 4,5,9, 13,14 | |
| | ----- | | |

TECHNICAL FIELDS SEARCHED (IPC)

A61B

The present search report has been drawn up for all claims

| Place of search | Date of completion of the search | Examiner |
|---|---|---|
| The Hague | 13 May 2026 | Costa Angeli, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

............................................................................................

& : member of the same patent family, corresponding document

EPO FORM 1503 03.82 (P04C01)

**EP 4 778 456 A1**

## ANNEX TO THE EUROPEAN SEARCH REPORT
## ON EUROPEAN PATENT APPLICATION NO.

EP 26 15 1413

This annex lists the patent family members relating to the patent documents cited in the above-mentioned European search report.
The members are as contained in the European Patent Office EDP file on
The European Patent Office is in no way liable for these particulars which are merely given for the purpose of information.

13-05-2026

| Patent document cited in search report | Publication date | Patent family member(s) | | Publication date |
|---|---|---|---|---|
| US 2022233235 A1 | 28-07-2022 | EP | 4280989 A1 | 29-11-2023 |
| | | US | 2022233235 A1 | 28-07-2022 |
| | | WO | 2022159665 A1 | 28-07-2022 |
| US 2023112251 A1 | 13-04-2023 | CN | 115944272 A | 11-04-2023 |
| | | EP | 4162866 A1 | 12-04-2023 |
| | | IL | 296926 A | 01-05-2023 |
| | | JP | 2023057063 A | 20-04-2023 |
| | | US | 2023112251 A1 | 13-04-2023 |
| US 2024325066 A1 | 03-10-2024 | EP | 4687731 A2 | 11-02-2026 |
| | | US | 2024325066 A1 | 03-10-2024 |
| | | WO | 2024206880 A2 | 03-10-2024 |

EPO FORM P0459

For more details about this annex : see Official Journal of the European Patent Office, No. 12/82